# EUROPEAN PATENT APPLICATION

(11) **EP 4 389 739 A1**
(43) Date of publication of application: **26.06.2024**
(21) Application number: 22215282.9
(22) Date of filing: 21.12.2022
(51) Int. Cl.: C07D 307/68, A01N 43/08

(54) **MOLECULE HAVING PESTICIDAL UTILITY AND PROCESSES RELATED THERETO**

(71) Applicant: Corteva Agriscience LLC, Indianapolis, IN 46268 (US)
(72) Inventor: BAIDOO, Richard, Indianopolis, Indiana 46240 (US); BANGEL, Bryston L., Mooresville, Indiana 46158 (US); LOSO, Michael R., Carmel, Indiana 46032 (US)
(74) Representative: f & e patent

(57) **Abstract**

This disclosure relates to the field of a molecule having pesticidal utility against pests in Phylum Nematoda, and processes of using such a molecule on such pests. This pesticidal molecule may be used in pesticidal compositions, such as, for example, nematicidal compositions.

## Description

### BACKGROUND FOR THIS DISCLOSURE

This disclosure relates to the field of a molecule having pesticidal utility against pests in Phylum Nematoda, and processes of using such a molecule on such pests. This pesticidal molecule may be used in pesticidal compositions, such as, for example, nematicidal compositions.

"Many of the most dangerous human diseases are transmitted by insect vectors" (Rivero et al., Insect Control of Vector-Borne Diseases: When is Insect Resistance a Problem? Public Library of Science Pathogens, Vol. 6, No. 8, p. 1-9, 2010). "Historically, malaria, dengue, yellow fever, plague, filariasis, louse-borne typhus, trypanosomiasis, leishmaniasis, and other vector borne diseases were responsible for more human disease and death in the 17^{th} through the early 20^{th} centuries than all other causes combined" (Gubler, D., Resurgent Vector-Borne Diseases as a Global Health Problem, Emerging Infectious Diseases, Vol. 4, No. 3, p. 442-450, 1998). Vector-borne diseases are responsible for about 17% of the global parasitic and infectious diseases. Malaria alone causes over 800,000 deaths a year, 85% of which occur in children under five years of age. Each year there are about 50 to about 100 million cases of dengue fever. A further 250,000 to 500,000 cases of dengue hemorrhagic fever occur each year (Matthews., Integrated Vector Management: Controlling Vectors of Malaria and Other Insect Vector Borne Diseases, Ch. 1, p. 1, 2011). Vector control plays a critical role in the prevention and control of infectious diseases. However, insecticide resistance, including resistance to multiple insecticides, has arisen in all insect species that are major vectors of human diseases (Rivero et al.). Recently, more than 550 arthropod species have developed resistance to at least one pesticide (Whalon et al. Analysis of Global Pesticide Resistance in Arthropods, Global Pesticide Resistance in Arthropods, Ch. 1, p. 5-33, 2008). Furthermore, the cases of insect resistance continue to exceed by far the number of cases of herbicide and fungicide resistance (Sparks et al., IRAC: Mode of action classification and insecticide resistance management, Pesticide Biochemistry and Physiology (2014) available online 4 December 2014).

Each year, insects, plant pathogens, and weeds destroy more than 40% of all food produced. This loss occurs despite the application of pesticides and the use of a wide array of non-chemical controls, such as crop rotation and biological controls. If just some of this food could be saved, it could be used to feed the more than three billion people in the world who are malnourished (Pimental, D., Pest Control in World Agriculture Agricultural Sciences - Vol. II, 2009).

Plant parasitic nematodes are among the most widespread pests and are frequently one of the most insidious and costly, causing an estimated annual yield loss of over $100 billion worldwide (Abad et al., Root-knot nematode parasitism and host response: Molecular basis of a sophisticated interaction. Mol. Plant Pathol. 4, 217-224. doi: 10.1046/j. 1364-3703.2003.00170.x). It has been estimated that losses attributable to nematodes range from about 9% in developed countries to about 15% in undeveloped countries. However, in the United States of America a survey of 35 States on various crops indicated nematode-derived losses of up to 25% (Nicol et al., Current Nematode Threats to World Agriculture, Genomic and Molecular Genetics of Plant - Nematode Interactions, p. 21-43, 2011).

About 2000 plants worldwide are susceptible to infection by root-knot nematodes and they cause approximately 5% of global crop loss (Haydock et al: Haydock PJ, Woods SR, Grove G, Hare MC. Chemical control of nematodes. In: Perry RN, Moens M, eds. Plant Nematology. Wallingford, Cambridge: CAB International; 2006:392-408. http://dx.doi.org/10.1079/9781845930561.0392 Accessed 14.11.16). Root-knot nematodes (RKN), *Meloidogyne spp.*, are one of the three most economically damaging genera of plant-parasitic nematodes in agriculture. Root-knot nematodes are distributed worldwide. The genus includes more than 90 species. Four Meloidogyne species (*M. javanica, M. arenaria, M. incognita, and M. hapla*) are major pests worldwide, with another seven being important on a local basis. If root-knot nematodes become established in deep-rooted, perennial crops, control is difficult, and options are limited.

The soybean cyst nematode (SCN), *Heterodera glycines*, is the most devastating pest to soybean crop yields in the United States with an estimated annual yield losses of more than $1 billion targeting the roots of soybean and other legume plants (Wrather et al: Soybean disease loss estimates for the top ten soybean producing countries in 1998. Canad. J. Plant Pathol. 23: 115-121). The symptoms caused by SCNs can go easily unrecognized by farmers - in some cases there are no warning symptoms before a loss of 30% of the yield.

Root lesion nematode aboveground symptoms on plants with severe root infection may include chlorosis, stunting, and low vigor. Infected roots may be poorly developed and bear small, brown-black lesions. Lesion nematodes are migratory endoparasites.

Consequently, for many reasons, including those mentioned above, there is an on-going need for the costly (estimated to be about US$286 million per pesticide in 2014), time-consuming (on average about 11.3 years per pesticide), and difficult development of new pesticides (Phillips McDougall, The Cost of New Agrochemical Product Discovery, Development and Registration in 1995, 2000, 2005-8 and 2010-2014. R&D expenditure in 2014 and expectations for 2019, 2016).

### DEFINITIONS FOR THIS DISCLOSURE

Examples provided herein are not exhaustive and should not be construed as limiting. It is understood that a substituent should comply with chemical bonding rules and steric compatibility constraints in relation to the particular molecule to which it is attached. These definitions are only to be used for the purposes of this disclosure.

The term "locus" means a habitat, breeding ground, plant, seed, soil, material, or environment, in which a pest is growing, may grow, or may traverse. For example, a locus may be: where crops, trees, fruits, cereals, fodder species, vines, turf, and/or ornamental plants, are growing; where domesticated animals are residing; the interior or exterior surfaces of buildings (such as places where grains are stored); the materials of construction used in buildings (such as impregnated wood); and the soil around buildings.

The term "pest" means an organism that is detrimental to humans or human concerns (such as, crops, food, livestock, etc.), where said organism is from Phylum Nematoda, such as, for example, and includes, but is not limited to, *Aphelenchoides* spp., *Belonolaimus* spp., *Criconemella* spp., *Ditylenchus* spp., *Globodera* spp., *Heterodera* spp., *Hirschmanniella* spp., *Hoplolaimus* spp., *Meloidogyne* spp., *Pratylenchus* spp., and *Radopholus* spp. A non-exhaustive list of particular species includes, but is not limited to, *Dirofilaria immitis*, *Globodera pallida*, *Heterodera glycines, Heterodera zeae, Meloidogyne incognita, Meloidogyne javanica, Onchocerca volvulus*, *Pratylenchus penetrans*, *Radopholus similis*, and *Rotylenchulus reniformis.*

The phrase "pesticidally effective amount" means the amount of a pesticide needed to achieve an observable effect on a pest, for example, the effects of necrosis, death, retardation, prevention, removal, destruction, or otherwise diminishing the occurrence and/or activity of a pest in a locus. This effect may come about when pest populations are repulsed from a locus, pests are incapacitated in or around a locus, and/or pests are exterminated in or around a locus. Of course, a combination of these effects can occur. Generally, pest populations, activity, or both are desirably reduced more than fifty percent, preferably more than 90 percent, and most preferably more than 99 percent. In general, a pesticidally effective amount, for agricultural purposes, is from about 0.0001 grams per hectare to about 5000 grams per hectare, preferably from about 0.0001 grams per hectare to about 500 grams per hectare, and it is even more preferably from about 0.0001 grams per hectare to about 50 grams per hectare.

All references, including publications, patent applications, and patents, referred to herein are incorporated by reference herein to the same extent as if each reference were individually and specifically indicated to be incorporated by reference and were set forth in its entirety.

The use of the terms "a", "an", "the", "at least one", and similar referents in the context of this disclosure (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples or exemplary language (e.g., "such as") provided herein is intended merely to better illuminate the disclosure and does not pose a limitation on the scope of the disclosure unless otherwise claimed. No language in the specification should be construed as indicating any non-claimed element as essential to the practice of any embodiment herein. In this disclosure the terms "molecule" and "compound" may be used interchangeably.

### DETAILED DESCRIPTION FOR THIS DISCLOSURE

This document discloses the following molecule.

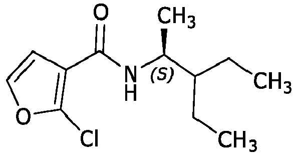

The chemical name of this molecule (*S*)-2-chloro-*N*-(3-ethylpentan-2-yl)furan-3-carboxamide. (For the purpose of this disclosure it is also known as "**S1**").

A variety of methods known in the art may be used to make this molecule, such as, for example, WO 2017116646, US 20200397000, WO 2007039615, and US 8148538. Additionally, various separation techniques are available to isolate specific enantiomers such as S1 from racemic mixtures such as B1. For example, chiral chromatography may be used to isolate enantiomers, as well as, using this method to determine enantiomeric purity (Journal of Pharmaceutical and Biomedical Analysis, 2013 and "Principles and Practice of Modern Chromatographic Methods (Second Edition), 2022).

### EXAMPLES

### Example One

S1 was compared to the following molecules. 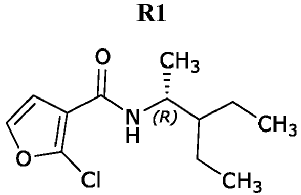 (*R*)-2-chloro-*N-*(3-ethylpentan-2-yl)furan-3-carboxamide; and 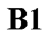 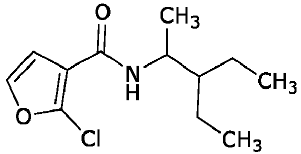 2-chloro-*N*-(3-ethylpentan-2-yl)furan-3-carboxamide (CAS REG No. 1876009-19-6) **B1** is a racemic mixture of S1 and R1, and S1 and R1 are enantiomers.

### Part One

The following Root Knot Nematode (RKN) test was conducted to compare the activities of S1, R1, and B1.

### Test procedure

We conducted an experiment to evaluate comparative bioactivity of S1, R1, and B1 against the root knot nematode (RKN), *Meloidogyne incognita.* Each of S1, R1, and B1 were tested at six rates: 125, 62.5, 31.25, 15.6, 7.8, 3.9, and 2 ppm. Cucumber seeds (*Cucumis sativus*) were planted in a sandy loam soil in 3-inch pots. After 6 days of germination, the seedlings were treated with the test compounds dissolved in 5% acetone in water. The treated plants were inoculated with 4,000 second stage juveniles (J2s) of *M. incognita.* Each experimental unit was replicated three times. The inoculated plants were kept in a Greenhouse for 14 days (about 27 °C) before evaluating the amount of root damage (galls) called Galling Index (GI) based on a scale of 0 - 10; where 0 = no galls (100% control), and 10 = maximum galls (no control). The GI was converted to % control using the formula (10 - GI) x 10; where GI = Gall Index. Effective concentration (EC) values based on the % control of the nematode by S1, R1 and B1 were calculated by probit analysis.

### Test Results and Conclusions.

| **Molecule** | **EC₅₀ (ppm)** | **Delta % vs R1*** | **Delta % vs B1** |
|---|---|---|---|
| **S1** | 4.95 | >2425 | 240 |
| **R1** | >125 | | |
| **B1** | 16.84 | | |
| EC is short for effective concentration | | | |
| * example ((125-4.95)/4.95)^{∗}100 | | | |

These data show that S1 is clearly and unexpectedly far superior to R1 and B 1 considering the magnitudes of the Delta percent (Delta %).

### Part Two

The following Soybean Cyst Nematode (SCN) test was conducted to compare the activities of S1, R1, and B1.

### Test procedure

We carried out an *in-vitro* experiment to compare activity of S1, R1, and B1 with each other against the soybean cyst nematode (SCN): *Heterodera glycines* second stage juveniles (J2). Each of S1, R1, and B1 were tested at six rates: 62.5, 15.6, 4, 1, 0.25, and 0 ppm (untreated control, UTC) with three replications of each treatment. Equal volume of nematode suspension was added to an equal volume of compound dissolved in 0.5% DMSO (dimethyl sulfoxide) in water. The nematode plus compound was incubated at room temperature (about 25 °C) on a shaker at 95 revolutions per minutes (RPM). Aliquots of 0.5 milliliters (mL) of the nematode suspension were evaluated under a microscope for mortality after 48 hours of incubation. SCN J2s were counted, and the number of dead and active nematodes was used to calculate % SCN mortality which was used to generate lethal concentration (LC) values by probit analysis.

### Test Results and Conclusions.

| **Molecule** | **LC₅₀ (ppm)** | **Delta % vs R1*** | **Delta % vs B1** |
|---|---|---|---|
| **S1** | 1.799 | >3374 | 1194 |
| **R1** | >62.5 | | |
| **B1** | 23.28 | | |
| LC is short for lethal concentration | | | |
| * example ((62.5-1.799)/1.799)*100 | | | |

These data show that S1 is clearly and unexpectedly far superior to R1 and B 1 considering the magnitudes of the Delta percent (Delta %).

### Part Three

The following Root Lesion Nematode (RLN) test was conducted to compare the activities of S1, R1, and B1.

### Test procedure

We carried out an *in-vitro* experiment to compare activity of S1, R1, and B1 with each other against the root lesion nematode (RLN), *Pratylechus penetrans.* Each of S1, R1, and B1 were tested at six rates: 62.5, 15.6, 4, 1, 0.25, and 0 ppm (UTC) with three replications of each treatment. Equal volume of nematode suspension was added to an equal volume of each of S1, R1, and B1 dissolved in 0.5% DMSO (dimethyl sulfoxide) in water in 25 mL vials. The treated nematode suspension was incubated at room temperature (about 25 °C) on a shaker (95 RPM). Aliquots of 1 mL of the nematode suspension was evaluated under a microscope for mortality after 48 hours. RLN were counted, and the number of dead and active nematodes was used to calculate % mortality from which lethal concentration (LC) values were generated.

### Test Results and Conclusions

| **Molecule** | **LC₅₀ (ppm)** | **Delta% vs R1*** | **Delta % vs B1** |
|---|---|---|---|
| **S1** | 6.89 | >807 | 259 |
| **R1** | >62.5 | | |
| **B1** | 24.72 | | |
| LC is short for lethal concentration | | | |
| * example ((62.5-6.89)/6.89)*100 | | | |

These data show that S1 is clearly and unexpectedly far superior to R1 and B 1 considering the magnitudes of the Delta percent (Delta %).

In summary, the experimental data are unexpected and surprising especially considering that, comparing S1 versus R1, the Delta % was >2202 on average, and comparing S1 versus B 1, the Delta % was 564 on average. Such magnitude of change prompted additional research.

### Example Two

The following molecules were compared.

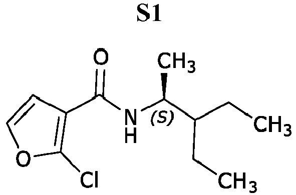

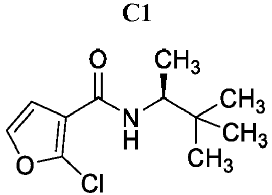

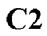

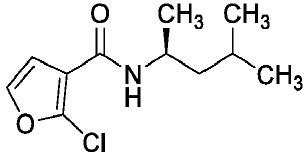

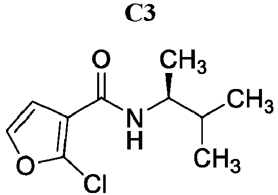

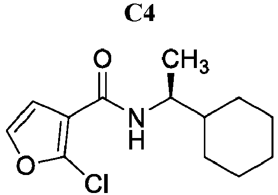

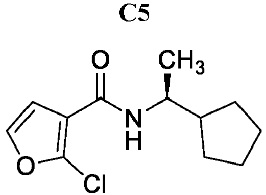

### Testing procedure

We carried out an experiment to evaluate comparative bioactivity of S 1, C1, C2, C3, C4, and C5 against the root knot nematode, *Meloidogyne incognita.* Each compound was tested at 600 grams of active ingredient per hectare (g ai/ha) and was replicated three times. Cucumber seeds (*Cucumis sativus*) were planted in a sandy loam soil in 3-inch pots. After six (6) days of planting, the seedlings were treated with the test compounds dissolved in 5% acetone in water by drench application. The plants were inoculated with 4,000 second stage juveniles (J2s) of *M*. *incognita.* The inoculated plants were kept in a Greenhouse for 14 days (temperature 27 °C) before evaluating the amount of root damage (galls) called Galling Index (GI) based on a scale of 0 - 10; where 0 = no galls (100% nematode control), and 10 = maximum galls (no nematode control). The GI was converted to % nematode control using the formula ((10 - GI) x 10); where GI = Gall Index. The data were analyzed by Analysis of Variance using JMP (John's Macintosh Project) statistical software and the means were separated using Tukey's HSD (α = 0.05).

### Test Results and Conclusions

| **Molecule** | **Statistical Group** | **Mean Percent Control** |
|---|---|---|
| **S1** | A | 84 |
| **C1** | B C | 54 |
| **C2** | B C | 54 |
| **C3** | B | 67 |
| **C4** | D | 25 |
| **C5** | C | 50 |
| **Control** | E | 0 |

The results show that only S1 has a level of control that is acceptable and statistically superior to the other molecules tested. **At the projected field rate of 600 g ai/ha, the threshold root knot nematode control is set at > = 75%.**

### Example Three

The molecules in Example Two were compared in a residuality test.

### Testing procedure

A residuality test was conducted to compare the residual control or persistence of S 1, R1, C1, C2, C3, C4, and C5 in soil. Each compound was applied to a bare sandy loam soil (without plant or nematode) in a 3-inch pot at 1250 g ai/ha. The treated soils were kept in a Greenhouse (temperature 27 °C) under regular irrigation for four weeks. At the end of the four weeks, six-day old cucumber (*Cucumis sativus*) seedlings were transplanted into the treated soil and inoculated with 4,000 second stage juveniles of *M. incognita.* Each treatment was replicated four times. The inoculated plants were kept in a Greenhouse for 14 days (temperature 27 °C) before evaluating the amount of root damage (galls) called Galling Index (GI) based on a scale of 0 - 10; where 0 = no galls (100% nematode control), and 10 = maximum galls (no nematode control). The GI were converted to % control using the formula ((10 - GI) x 10); where GI = Gall Index. The data were analyzed by Analysis of Variance using JMP (John's Macintosh Project) statistical software and the means were separated using Tukey's HSD (α = 0.05).

### Test Results and Conclusions

| **Molecule** | **Statistical Group** | **Mean Percent Control** |
|---|---|---|
| **S1** | A | 77.5 |
| **R1** | C | 7.5 |
| **C1** | B | 27.5 |
| **C2** | B | 25.0 |
| **C3** | B | 22.5 |
| **C4** | C | 2.5 |
| **C5** | B | 22.5 |
| **Control** | C | 2.5 |

The results show that only S1 has a surprising and unexpected level of residuality when compared to the other molecules. In summary, based on the above, S1 has unexpected and surprising properties that are statistically far superior to the other molecules.

A composition comprising S1 may be made by techniques known in the art. A currently preferred example is a nematicide composition that comprises S1 as the only nematicidal active. However, other actives may also be present, such as, for example, R1. If R1 and S1 are present in a composition, it is preferred that the composition is enantiomerically enriched with S1; in other words, the enantiomeric ratio of S1 to R1 is greater than 50:50 but less than 100:0 (see "enantiomerically enriched" IUPAC. Compendium of Chemical Terminology, 2nd ed. (the "Gold Book"). Compiled by A. D. McNaught and A. Wilkinson. Blackwell Scientific Publications, Oxford (1997). Online version (2019-) created by S. J. Chalk. ISBN 0-9678550-9-8. https://doi.org/10.1351/goldbook). Generally, depending on the production process, an enantiomeric ratio of S1 to R1 greater than 90:10 is preferred, an enantiomeric ratio of S1 to R1 greater than 95:5 is more preferred, and an enantiomeric ratio of S1 to R1 greater than greater than 97:3 is even more preferred. However, in some situations where a composition is going to be used in a particular environment an enantiomeric ratio of S1 to R1 of 100:0 is preferred.

The following additional details are provided.
**1d.** The molecule (*S*)-2-chloro-*N*-(3-ethylpentan-2-yl)furan-3-carboxamide (hereinafter "SI") 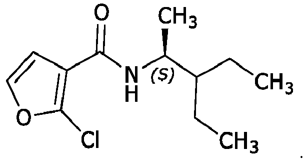
**2d.** A composition comprising:
   (a) the molecule according to detail 1d; but not
   (b) (*R*)-2-chloro-*N*-(3-ethylpentan-2-yl)furan-3-carboxamide (hereinafter "R1") 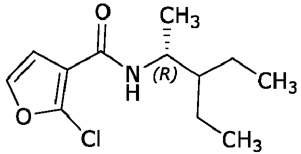
   in other words, the enantiomeric ratio of S1 to R1 is 100:0.
**3d.** A composition comprising:
   (a) the molecule according to detail 1d (hereinafter "S1"); and
   (b) (*R*)-2-chloro-*N*-(3-ethylpentan-2-yl)furan-3-carboxamide (hereinafter "R1");
   wherein the enantiomeric ratio of S1 to R1 is greater than 50:50 but less than 100:0.
**4d.** A composition according to detail **3d** wherein the enantiomeric ratio of S1 to R1 is greater than 51:49 but less than 100:0.
**5d.** A composition according to detail **3d** wherein the enantiomeric ratio of S1 to R1 is greater than 52:48 but less than 100:0.
**6d.** A composition according to detail **3d** wherein the enantiomeric ratio of S1 to R1 is greater than 53:47 but less than 100:0.
**7d.** A composition according to detail **3d** wherein the enantiomeric ratio of S1 to R1 is greater than 54:46 but less than 100:0.
**8d.** A composition according to detail **3d** wherein the enantiomeric ratio of S1 to R1 is greater than 55:45 but less than 100:0.
**9d.** A composition according to detail **3d** wherein the enantiomeric ratio of S1 to R1 is greater than 56:44 but less than 100:0.
**10d.** A composition according to detail **3d** wherein the enantiomeric ratio of S1 to R1 is greater than 57:43 but less than 100:0.
**11d.** A composition according to detail **3d** wherein the enantiomeric ratio of S1 to R1 is greater than 58:42 but less than 100:0.
**12d.** A composition according to detail **3d** wherein the enantiomeric ratio of S1 to R1 is greater than 59:41 but less than 100:0.
**13d.** A composition according to detail **3d** wherein the enantiomeric ratio of S1 to R1 is greater than 60:40 but less than 100:0.
**14d.** A composition according to detail **3d** wherein the enantiomeric ratio of S1 to R1 is greater than 61:39 but less than 100:0.
**15d.** A composition according to detail **3d** wherein the enantiomeric ratio of S1 to R1 is greater than 62:38 but less than 100:0.
**16d.** A composition according to detail **3d** wherein the enantiomeric ratio of S1 to R1 is greater than 63:37 but less than 100:0.
**17d.** A composition according to detail **3d** wherein the enantiomeric ratio of S1 to R1 is greater than 64:36 but less than 100:0.
**18d.** A composition according to detail **3d** wherein the enantiomeric ratio of S1 to R1 is greater than 65:35 but less than 100:0.
**19d.** A composition according to detail **3d** wherein the enantiomeric ratio of S1 to R1 is greater than 66:34 but less than 100:0.
**20d.** A composition according to detail **3d** wherein the enantiomeric ratio of S1 to R1 is greater than 67:33 but less than 100:0.
**21d.** A composition according to detail **3d** wherein the enantiomeric ratio of S1 to R1 is greater than 68:32 but less than 100:0.
**22d.** A composition according to detail **3d** wherein the enantiomeric ratio of S1 to R1 is greater than 69:31 but less than 100:0.
**23d.** A composition according to detail **3d** wherein the enantiomeric ratio of S1 to R1 is greater than 70:30 but less than 100:0.
**24d.** A composition according to detail **3d** wherein the enantiomeric ratio of S1 to R1 is greater than 71:29 but less than 100:0.
**25d.** A composition according to detail **3d** wherein the enantiomeric ratio of S1 to R1 is greater than 72:28 but less than 100:0.
**26d.** A composition according to detail **3d** wherein the enantiomeric ratio of S1 to R1 is greater than 73:27 but less than 100:0.
**27d.** A composition according to detail **3d** wherein the enantiomeric ratio of S1 to R1 is greater than 74:26 but less than 100:0.
**28d.** A composition according to detail **3d** wherein the enantiomeric ratio of S1 to R1 is greater than 75:25 but less than 100:0.
**29d.** A composition according to detail **3d** wherein the enantiomeric ratio of S1 to R1 is greater than 76:24 but less than 100:0.
**30d.** A composition according to detail **3d** wherein the enantiomeric ratio of S1 to R1 is greater than 77:23 but less than 100:0.
**31d.** A composition according to detail **3d** wherein the enantiomeric ratio of S1 to R1 is greater than 78:22 but less than 100:0.
**32d.** A composition according to detail 3d wherein the enantiomeric ratio of S1 to R1 is greater than 79:21 but less than 100:0.
**33d.** A composition according to detail **3d** wherein the enantiomeric ratio of S1 to R1 is greater than 80:20 but less than 100:0.
**34d.** A composition according to detail **3d** wherein the enantiomeric ratio of S1 to R1 is greater than 81:19 but less than 100:0.
**35d.** A composition according to detail **3d** wherein the enantiomeric ratio of S1 to R1 is greater than 82:18 but less than 100:0.
**36d.** A composition according to detail **3d** wherein the enantiomeric ratio of S1 to R1 is greater than 83:17 but less than 100:0.
**37d.** A composition according to detail **3d** wherein the enantiomeric ratio of S1 to R1 is greater than 84:16 but less than 100:0.
**38d.** A composition according to detail **3d** wherein the enantiomeric ratio of S1 to R1 is greater than 85:15 but less than 100:0.
**39d.** A composition according to detail **3d** wherein the enantiomeric ratio of S1 to R1 is greater than 86:14 but less than 100:0.
**40d.** A composition according to detail **3d** wherein the enantiomeric ratio of S1 to R1 is greater than 87:13 but less than 100:0.
**41d.** A composition according to detail **3d** wherein the enantiomeric ratio of S1 to R1 is greater than 88:12 but less than 100:0.
**42d.** A composition according to detail **3d** wherein the enantiomeric ratio of S1 to R1 is greater than 89:11 but less than 100:0.
**43d.** A composition according to detail **3d** wherein the enantiomeric ratio of S1 to R1 is greater than 90:10 but less than 100:0.
**44d.** A composition according to detail **3d** wherein the enantiomeric ratio of S1 to R1 is greater than 91:9 but less than 100:0.
**45d.** A composition according to detail **3d** wherein the enantiomeric ratio of S1 to R1 is greater than 92:8 but less than 100:0.
**46d.** A composition according to detail **3d** wherein the enantiomeric ratio of S1 to R1 is greater than 93:7 but less than 100:0.
**47d.** A composition according to detail **3d** wherein the enantiomeric ratio of S1 to R1 is greater than 94:6 but less than 100:0.
**48d.** A composition according to detail **3d** wherein the enantiomeric ratio of S1 to R1 is greater than 95:5 but less than 100:00
**49d.** A composition according to detail **3d** wherein the enantiomeric ratio of S1 to R1 is greater than 96:4 but less than 100:0.
**50d.** A composition according to detail **3d** wherein the enantiomeric ratio of S1 to R1 is greater than 97:3 but less than 100:0.
**51d.** A composition according to detail **3d** wherein the enantiomeric ratio of S1 to R1 is greater than 98:2 but less than 100:0.
**52d.** A composition according to detail **3d** wherein the enantiomeric ratio of S1 to R1 is greater than 99:1 but less than 100:0.
**53d.** A process to control a pest in a locus by using a pesticidally effective amount of
   **(a)** the molecule according to detail 1d; or
   **(b)** a composition according to one of details 2d, 3d, 4d, 5d, 6d, 7d, 8d, 9d, 10d, 11d, 12d, 13d, 14d, 15d, 16d, 17d, 18d, 19d, 20d, 21d, 22d, 23d, 24d, 25d, 26d, 27d, 28d, 29d, 30d, 31d, 32d, 33d, 34d, 35d, 36d, 37d, 38d, 39d, 40d, 41d, 42d, 43d, 44d, 45d, 46d, 47d, 48d, 49d, 50d, 51d, or 52d.
**54d.** Use of the molecule according to detail 1d; or the composition according to any of according to one of details 2d, 3d, 4d, 5d, 6d, 7d, 8d, 9d, 10d, 11d, 12d, 13d, 14d, 15d, 16d, 17d, 18d, 19d, 20d, 21d, 22d, 23d, 24d, 25d, 26d, 27d, 28d, 29d, 30d, 31d, 32d, 33d, 34d, 35d, 36d, 37d, 38d, 39d, 40d, 41d, 42d, 43d, 44d, 45d, 46d, 47d, 48d, 49d, 50d, 51d, or 52d to control a pest from Phylum Nematoda.

**The claims follow.**

## Claims

1. The molecule (*S*)-2-chloro-*N*-(3-ethylpentan-2-yl)furan-3-carboxamide

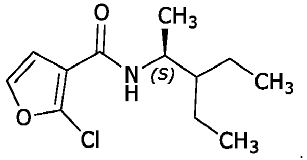

2. A composition comprising:
**(a)** the molecule according to claim 1; but not
**(b)** (*R*)-2-chloro-*N*-(3-ethylpentan-2-yl)furan-3-carboxamide (**hereinafter "R1"**)

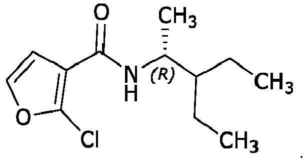

3. A composition comprising:
**(a)** the molecule according to claim 1 (**hereinafter "S1"**); and
**(b)** (*R*)-2-chloro-*N*-(3-ethylpentan-2-yl)furan-3-carboxamide (**hereinafter "R1"**); wherein the enantiomeric ratio of S1 to R1 is greater than 50:50 but less than 100:0.

4. A composition according to claim **3** wherein the enantiomeric ratio of S1 to R1 is greater than 90: 10 but less than 100:0.

5. A composition according to claim **3** wherein the enantiomeric ratio of S1 to R1 is greater than 91:9 but less than 100:0.

6. A composition according to claim **3** wherein the enantiomeric ratio of S1 to R1 is greater than 92:8 but less than 100:0.

7. A composition according to claim **3** wherein the enantiomeric ratio of S1 to R1 is greater than 93:7 but less than 100:0.

8. A composition according to claim **3** wherein the enantiomeric ratio of S1 to R1 is greater than 94:6 but less than 100:0.

9. A composition according to claim **3** wherein the enantiomeric ratio of S1 to R1 is greater than 95:5 but less than 100:0.

10. A composition according to claim **3** wherein the enantiomeric ratio of S1 to R1 is greater than 96:4 but less than 100:0.

11. A composition according to claim **3** wherein the enantiomeric ratio of S1 to R1 is greater than 97:3 but less than 100:0.

12. A composition according to claim **3** wherein the enantiomeric ratio of S1 to R1 is greater than 98:2 but less than 100:0.

13. A composition according to claim **3** wherein the enantiomeric ratio of S1 to R1 is greater than 99:1 but less than 100:0.

14. A process to control a pest in a locus by using a pesticidally effective amount of
**(a)** the molecule according to claim 1; or
**(b)** a composition according to any of claims 2, 3, 4, 5, 6, 7, 8, 9, 10, 11, 12, or 13.

15. Use of the molecule according to claim 1; or the composition according to any of claims 2, 3, 4, 5, 6, 7, 8, 9, 10, 11, 12, or 13 to control a pest from Phylum Nematoda.
